# EUROPÄISCHE PATENTSCHRIFT

(11) **EP 3 474 953 B1**
(45) Veröffentlichungstag und Bekanntmachung des Hinweises auf die Patenterteilung: **16.11.2022**
(21) Anmeldenummer: 17734041.1
(22) Anmeldetag: 23.06.2017
(51) Int. Cl.: A61Q 19/00, A61Q 19/08, A61K 8/9783

(54) **GLYKOSAMINOGLYKAN ENTHALTENES STOFFGEMISCH SOWIE EIN VERFAHREN ZU DESSEN HERSTELLUNG UND DESSEN VERWENDUNG**
SUBSTANCE MIXTURE CONTAINING GLYCOSAMINOGLYCAN, METHOD FOR PRODUCING SAME AND USE OF SAME
MÉLANGE DE SUBSTANCES CONTENANT UN GLYCOSAMINOGLYCANE, ET PROCÉDÉ DE PRODUCTION ET UTILISATION DUDIT MÉLANGE

(30) Priorität: 24.06.2016 DE 102016111592
(43) Veröffentlichungstag der Anmeldung: 01.05.2019
(73) Patentinhaber: Fach, Dr. Christian, 40549 Düsseldorf (DE)
(72) Erfinder: Fach, Dr. Christian, 40549 Düsseldorf (DE)
(74) Vertreter: Bals & Vogel Patentanwälte PartGmbB
(86) Internationale Anmeldenummer: PCT/EP2017/065576
(87) Internationale Veröffentlichungsnummer: WO 2017/220796

(56) Entgegenhaltungen:
- WO-A2-2005/000248
- WO-A2-2008/028097
- DE-A1-102009 008 940
- JP-A- 2006 111 630
- KR-A- 20150 007 530
- WANG FRANK ET AL: "In vivo stimulation of de novo collagen production caused by cross-linked hyaluronic acid dermal filler injections in photodamaged human skin", ARCHIVES OF DERMATOLOGY, AMERICAN MEDICAL ASSOCIATION, US, vol. 143, no. 2, 1 February 2007 (2007-02-01), pages 155-163, XP002574140, ISSN: 0003-987X, DOI: 10.1001/ARCHDERM.143.2.155

## Beschreibung

Die vorliegende Erfindung betrifft ein Verfahren zur Behandlung der Haut, umfassend ein Glykosaminoglykan enthaltenes Stoffgemisch gemäß dem unabhängigen Patentanspruch 1 sowie ein Verfahren zur Herstellung des Stoffgemisches gemäß dem unabhängigen Anspruch 5 und die Verwendung des Glykosaminoglykan enthaltenen Stoffgemisches gemäß dem unabhängigen Patentanspruch 6.

Glykosaminoglykan enthaltene Stoffgemische sind im Stand der Technik bekannt. Beispielsweise offenbart die Schrift DE 10 2009 008 940 A1 eine Kombination von Hyaluronsäuregelen und Cremes in separater Zubereitung als Produkt zur Körper- und Schönheitspflege. Dabei hat sich der Nachteil herausgestellt, dass Glykosaminoglykane zwar einen kurzzeitigen positiven Effekt auf die Alterung der Haut besitzen, diesen Prozess jedoch nicht rückgängig machen können.

Die Schrift KR2015/0007530 A offenbart eine Feuchtigkeitscreme für die Haut, die gegen Trockenheit wirkt und die Elastizität der Haut erhöht. Die Schrift JP2066111630 A offenbart ein kosmetisches Stoffgemisch zur Unterstützung der Feuchtigkeit der Haut.

Daher ist es die Aufgabe der vorliegenden Erfindung, ein Stoffgemisch sowie ein Verfahren zu dessen Herstellung und eine Verwendung des Stoffgemisches bereitzustellen, die die vorgenannten Nachteile zumindest teilweise beheben. Insbesondere ist es die Aufgabe der vorliegenden Erfindung, gleichzeitig mehrere Zeichen der Hautalterung zu vermeiden.

Zur Lösung dieser Aufgabe wird ein Verfahren zur Behandlung der Haut, umfassend ein Glykosaminoglykan enthaltenes Stoffgemisch mit den technischen Merkmalen des Patentanspruches 1 sowie ein Verfahren zur Herstellung eines Glykosaminoglykan enthaltenen Stoffgemisches mit den Merkmalen des Anspruches 5 und die Verwendung eines Glykosaminoglykan enthaltenen Stoffgemisches mit den Merkmalen des Anspruches 6 vorgeschlagen, denen nachfolgende besondere Bedeutung zukommt. Dabei gelten alle technischen Merkmale, die für das erfindungsgemäße Glykosaminoglykan enthaltene Stoffgemisch offenbart werden, auch für das erfindungsgemäße Verfahren zur Herstellung eines Glykosaminoglykan enthaltenen Stoffgemisches sowie zu der Verwendung eines Glykosaminoglykan enthaltenen Stoffgemisches und umgekehrt, sodass diesbezüglich jeweils wechselseitig Bezug genommen wird bzw. werden kann. Zweckmäßige Ausgestaltungen der Erfindung sind in den abhängigen Unteransprüchen aufgeführt.

Erfindungsgemäß handelt es sich um ein Verfahren zur Behandlung der Haut, umfassend ein Glykosaminoglykan enthaltenes Stoffgemisch, welches in einem Verfahren für die dermatologische Behandlung der Haut eingesetzt wird. Dabei ist in dem Stoffgemisch ebenfalls Astragalosid IV, insbesondere als hochreines Extrakt der Astragalus Membranaceus Wurzel, enthalten. Das Glykosaminoglykan kommt in chemisch identischer Form in Geweben und in Körperflüssigkeiten aller Wirbeltiere vor. Beim Menschen befindet sich der größte Teil in der Haut, insbesondere in den Hautschichten Dermis und Epidermis. Der biologische Abbau kann systematisch durch Enzyme, insbesondere Hyaluronidasen erfolgen. Die Stabilität der Glykosaminoglykane ist organabhängig und beträgt z. B. für die Haut bis zu 24 Std. Glykosaminoglykan gehört zu den primären Biomolekülen und zeichnet sich entsprechend durch hohe Wasserlöslichkeit und durch besondere hygroskopische Eigenschaften aus (1 g Glykosaminoglykan bindet bis zu 6000 g Wasser). Dies kann der Haut ihre Spannkraft und Elastizität verleihen. In der Haut kann Glykosaminoglykan zusätzlich als Radikalempfänger und Antioxidans wirken. Mit zunehmendem Alter verringert sich die Glykosaminoglykanmenge der Haut, die vom Körper selbstständig produziert wird, wodurch die Haut an Spannkraft und Elastizität verliert. Glykosaminoglykan kann daher Anwendung im Bereich der Augenheilkunde, Rheumatologie und Wundheilung als auch für kosmetische Zwecke finden. Durch die ausgeprägte hygroskopische Kapazität können die Glykosaminoglykane ein unmittelbar sichtbares hautstraffendes und glättendes Hautbild hervorrufen. Ferner ist erfindungsgemäß Astragalosid IV in dem Stoffgemisch enthalten. Astragalosid IV kann dabei vorteilhafterweise der Zellerneuerung dienen. Limitiert wird das Wachstumspotential von Zellen hauptsächlich durch die so genannten Telomere, die die Enden der linearen Chromosomen bilden. Telomere haben keine genetische Funktion, sie sind einfache DNA-Abschnitte (Wiederholungen von Basenpaaren), die den Rest des Chromosoms schützen können. Telomere bestehen bei Wirbeltieren aus einer hexamerischen DNA-Komponente mit sich wiederholender Basensequenz (TTAGGG), die als Schutzkappen ("capping function") an den Enden eines jeden Chromosoms liegen. Bei jeder Zellteilung verkürzen sich die Telomere der Tochterzellen, wodurch bei jeder Zellteilung genetische Informationen verloren gehen (Endreplikationsproblem), bis schließlich eine kritische Telomerlänge erreicht wird (das sogenannte "Hayflick-limit"). Nach einer bestimmten Anzahl an Zellteilungen wird eine kritische Telomerlänge erreicht, die eine ausreichende Protektion der Chromosomenenden nicht mehr zulässt. Die Zellen können sich nicht mehr teilen, gehen in das Stadium der Seneszenz über und verlieren großteils ihre funktionellen Eigenschaften. Die Länge der Telomere kann somit die replikative Alterung jeder somatischen Körperzelle reflektieren bzw. limitieren ("mitotische Uhr"). Eine dermatologische Behandlung der Haut mit einer Kombination von Glykosaminoglykan und Astragalosid IV kann ein Eindringen von Wirkstoffen durch das Stratum Corneum bis in tiefere Hautschichten bewirken. Astragalosid IV kann in der Kutis auf die dort ansässigen Zellen insbesondere Fibroblasten der Haut durch Aktivierung des Enzyms Telomerase wirken. Telomerase ist ein Enzym des Zellkerns, welches Endstücke der Chromosomen, sogenannte Telomere, durch ein Hinzufügen von Nukleotiden wiederaufbaut. Telomerase ist dabei eine reverse Transkriptase, in welcher der RNA-Anteil als Matrize verwendet wird. Durch die konsekutive Verlängerung der Telomere kann Astragalosid IV der individuellen, genetisch festgelegten Zellalterung entgegenwirken. Damit handelt es sich um ein kombiniertes Stoffgemisch zur dermatologischen Behandlung der Haut, welches einerseits der Haut mehr Elastizität bzw. Spannkraft verleihen kann und andererseits die Zellerneuerung fördern kann. Damit kann sowohl der Hautalterung bedingt durch Umwelteinflüsse als auch gleichzeitig der Hautalterung durch genetische Faktoren entgegengewirkt werden.

Es ist ebenfalls denkbar, dass eine intrinsische und/oder eine extrinsische Hautalterung vermeidbar ist. Die extrinsische Hautalterung (Umweltalterung) kann durch äußere Einflüsse wie z. B. UV-Strahlung, Nikotinabusus usw. hervorgerufen werden. Die intrinsische Hautalterung (Zeitalterung) kann die natürliche, genetisch determinierte Hautalterung darstellen. Intrinsische strukturelle Veränderungen der Haut können insbesondere Folgen einer natürlich genetisch determinierten Hautalterung sein. Neben den genetischen Einflüssen können weitere Faktoren wie beispielsweise der natürliche Hormonhaushalt und prooxidative Stoffwechselvorgänge für die intrinsische Hautalterung verantwortlich sein. Die intrinsische Hautalterung kann dabei durch eine verminderte Zellteilungsfähigkeit der Fibroblasten hervorgerufen werden. Diese verminderte Zellfähigkeit kann mit dem Abbau der Telomere, die die Enden mit den linearen Chromosomen bilden, zusammenhängen (Hayflick-Phänomen). Eine Aktivierung der Telomerase der am Hautaufbau beteiligten Zellen kann daher eine innovative Strategie bieten, Veränderungen der Hautstruktur und daraus resultierende Funktionsstörungen entgegen zu wirken. Eine der bekanntesten natürlichen Telomerase-Aktivatoren ist das sogenannte Astragalosid IV. Astragaloside gelten in der Wissenschaft als die wichtigsten Wirkstoffe der Heilpflanze Astragalus membranaceus. Die Wurzel von Astragalus membranaceus gehört zu den 50 wichtigsten Arzneimitteln der traditionellen chinesischen Medizin und wird dort unter dem Namen Huang Qi ( 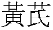 ) geführt. Astragalus ist eine Pflanzengattung der Unterfamilie der Schmetterlingsblütler (Faboideae), innerhalb der Familie der Hülsenfrüchtler. Mit etwa 1 600 bis 3 000 Arten ist sie die größte Gattung innerhalb der Gefäßpflanzen. Die mehrjährige Pflanze mit einer Höhe von 30 bis 80 cm wächst in China, der Mongolei, Sibirien und Korea auf trockenen Sandböden, Wiesen und gebirgigen Gegenden zwischen 800 bis 2 000 m. Astragalus membranaceus enthält mehr als 100 Inhaltsstoffe, insbesondere Polysaccharide (Mehrfachzucker), Saponine (Untergruppe der Tripernoiden) und Flavonoide. Astragalosid IV ist ein 3-0-beta-D-glucopyranosyl-cycloastragenol, tetrazyklisches Triterpenoid-Saponin. Saponine sind in höheren Pflanzen weit verbreitet, besonders in nährstoffreichem Gewebe, wie Wurzeln, Knollen, Blättern, Blüten und Samen. Sie dienen den Pflanzen als Defensivstoffe (Ersatz für Immunsystem), beispielsweise gegen Pilzbefall und Insektenfraß. Der molekulare Aufbau von Astragalosid IV ist C14H68O14. Das natürliche Glykosid hat ein hohes Molekulargewicht und damit insgesamt eine eingeschränkte intestinale Resorbierbarkeit. Die orale Bioverfügbarkeit beträgt 7,4 %. Die Bindung an Plasmaprotein A ist mit 90 % sehr hoch. Aufgrund des chemischen Aufbaus sind die Astragaloside stark lipophil. Es gibt verschiedene Extraktionsformen um Astragalosid IV in hohem Reinheitsgrad zu erhalten: Ultrafiltration, Highspeed-Zentrifugation, Extraktion mittels Ultraschall, Alkohol-Präzipitation. Astragalosid IV ist einer der bekanntesten Telomerase-Aktivatoren. Dabei kann der Zusatz von Astragalosid IV eine signifikante Telomer-Verlängerung bei kultivierten Fibroblasten induzieren. Die topische Applikation einer Kombination von Astragalosid IV und Glykosaminoglykan kann ein Eindringen der Substanzen durch das Stratum Corneum in tiefere Hautschichten bewirken.Insbesondere durch eine ausgeprägte hygroskopische Kapazität können Fragmente von Glykosaminoglykanen ein unmittelbar sichtbares, hautstraffendes und glättendes Hautbild hervorrufen. Dies geschieht in Abhängigkeit der Fragmentierung und des Molekulargewichts von Glykosaminoglykan. Darüber hinaus kann Glykosaminoglykan ein bekanntes transdermales Transportvehikel für Wirkstoffe und Biomakromoleküle in tiefere Hautschichten darstellen. Aufgrund einer negativen Ladung kann Glykosaminoglykan Proteine unter physiologischen Bedingungen binden und stabilisieren und einen Cotransport auch von großen Molekülen durch das Stratum Corneum bewirken. Astragalosid IV kann in der Kutis auf die dort ansässigen Zelllinien wirken, insbesondere durch Aktivierung der Telomerase. Durch die konsekutive Verlängerung der Telomere kann Astragalosid IV der individuellen, genetisch festgelegten Zellalterung entgegenwirken. Behandelte Zellen agieren vorzugsweise mit einem jüngeren Phänotyp. Somit kann das erfindungsgemäße Stoffgemisch eine kombinierte Hautpflege darstellen, die vorteilhaft sowohl auf die intrinsische als auch die extrinsische Hautalterung einwirken kann. Ferner weist das Glykosaminoglykan im Wesentlichen Hyaluronsäure auf. Bei Hyaluronsäuren kann es sich um Hyaluronsäure in reiner Form oder auch als ein Gel handeln, wobei Hyaluronsäure als kurzkettige Hyaluronsäure und/oder als langkettige Hyaluronsäure vorkommen kann. Ebenfalls ist eine Mischung aus kurz- und langkettigen Hyaluronsäuren denkbar. Vorliegend handelt es sich um Cross-Chain-Hyaluronketten, die eine Vernetzung aus langkettigen und kurzkettigen Hyaluronsäuren darstellen. Die Stabilität und damit Wirksamkeit einer Hyaluronsäure im Körper, insbesondere in der Haut, kann bei langkettigen und kurzkettigen Hyaluronsäuren ungefähr 24 Std. betragen. Bei Cross-Chain-Hyaluronsäuren kann diese Zeitspanne um bis zu 24 Std., also auf bis zu 48 Std. verlängert werden. Eine längere Stabilität und damit Wirksamkeit in der Haut kann somit gewährleistet werden. Hyaluronsäuren sind Glykosaminoglykane, makromolekulare Ketten, die sich aus wiederholenden Disaccharideinheiten zusammensetzen. Die Disaccharideinheiten bestehen aus über β-1,3-glykosidische-Bindungen miteinander verknüpfte D-Glucuronsäure und N-Acetylglucosamin. Die Disaccharideinheiten sind ferner über β-1,3-glykosidische-Bindungen zwischen dem N-Acetylglucosamin einer Disaccharideinheit und der D-Glucuronsäure der nächsten Disaccharideinheit miteinander verbunden. Typischerweise können ungefähr 250 Disaccharideinheiten bis ungefähr 50 000 Disaccharideinheiten eine Hyaluronsäurekette darstellen. Unter kurzkettigen Hyaluronsäuren können Hyaluronsäurenfraktionen von ungefähr 8 kDa bis ungefähr 100 kDa Molekulargewicht verstanden werden, bevorzugt von ungefähr 30 kDa bis ungefähr 70 kDa und besonders bevorzugt von ungefähr 40 kDa bis ungefähr 60 kDa. Unter langkettigen Hyaluronsäuren können Hyaluronsäurefraktionen von ungefähr 100 kDa bis ungefähr 5 000 kDa Molekulargewicht verstanden werden, bevorzugt von ungefähr 800 kDa bis ungefähr 2 000 kDa und besonders bevorzugt ungefähr 1 000 kDa bis ungefähr 2 000 kDa. Ferner ist es von Vorteil, wenn die Hyaluronsäuren fluidlöslich, insbesondere wasserlöslich sind. Ebenfalls ist es denkbar, dass auch die weiteren Bestandteile des Stoffgemisches fluidlöslich, insbesondere wasserlöslich sind.

Falls ein Wirkstoff wenig hydrophil oder sogar lipophil verwendet wird, ist es denkbar, dass die Löslichkeit durch liposomale Verkapselung, also Einbau in Lecithinliposomen verbessert werden kann beziehungsweise die Lösung in mittelkettigen Triglyzeriden (MCTs) erfolgen kann. Ein Eindringen der Hyaluronsäure an sich sowie die Funktion als Transportvehikel zum Eindringen anderer Substanzen, wie insbesondere Astragalosid IV, in die Haut kann somit gewährleistet werden.

Ferner ist es bei einem erfindungsgemäßen Stoffgemisch von Vorteil, dass das Glykosaminoglykan eine Konzentration von ungefähr 0,2 Gew.-% bis ungefähr 5 Gew.-%, bevorzugt eine Konzentration von ungefähr 0,5 Gew.-% bis ungefähr 4 Gew.-% und besonders bevorzugt eine Konzentration von ungefähr 1 Gew.-% bis ungefähr 2 Gew.-% aufweist. Gemäß einem weiteren Aspekt der Erfindung ist es vorteilhaft, dass das Astragalosid IV eine Konzentration von ungefähr 0,1 Gew.-% bis ungefähr 5 Gew.-%, bevorzugt eine Konzentration von ungefähr 0,1 Gew.-% bis ungefähr 4 Gew.-% und besonders bevorzugt eine Konzentration von ungefähr 0,1 Gew.-% aufweist. Eine derartige Konzentration von Glykosaminoglykan kann dabei ausreichend sein, sowohl eine hautstraffende Funktion auszuüben, als auch das Astragalosid IV zu binden und zu stabilisieren. Ein Transport bzw. Cotransport des Astragalosid IV mittels des Glykosaminoglykans durch das Stratum Corneum in die Kutis kann somit gewährleistet werden. Ferner kann durch eine limitierte Konzentration eine Kosteneffizienz gewährleistet werden. Die Konzentration des Astragalosid IV ist ferner ausreichend, um eine Telomerase-Aktivität hervorzurufen. Eine effektive Limitierung des Abbaus der Telomere wird dadurch erreicht. Insbesondere eine Konzentration von ungefähr 1 Gew.-% bis ungefähr 2 Gew.-% Glykosaminoglykan sowie insbesondere eine Konzentration von ungefähr 0,1 Gew.-% Astragalosid IV kann der Sättigung der Substanzen in der Haut, insbesondere aufgrund von Aufnahmekapazitäten der Haut sowie Bindekapazitäten am Wirkort, entsprechen. Ein Stoffgemisch kann somit mit geringem Aufwand und kosteneffizient hergestellt und verarbeitet werden.

Im Rahmen der Erfindung kann es ebenfalls vorteilhaft sein, wenn das Stoffgemisch zumindest eines der folgenden weiteren Additive aufweist: Paraffin, Glycerid, pflanzliches Fett, Kakaobutter, Mandelöl, Erdnussöl, Rizinusöl, Sojaöl, Wollwachs, Bienenwachs, Polysorbat, Macrogolether, Fettalkoholsulfat, Eucerit, Sorbitanfettsäureester, Monoglycerid, Sorbinsäure, Paraben, Butylhydroxytoluol, α-Tocopherol, Resveratrol, Vitamin-A-Säure, insbesondere Retinol und/oder Retinolsäure, Ascorbinsäure-2-Phosphat, Vitamin E, Tocopheryl Acetate, Vitamin D3, Vitamin B6/B12, Vitamin B3, Folsäure, Acetyl-L-Carnitin, Alpha-Liponsäure, Carnosin, Palmitoyl-Lysin-Threonin-Threonin-Lysin-Serin (pal-KTTS), 2-Dimethylaminoethanol (DMAE), Coenzym Q 10, Carnitin, Curcumin, Aloe Vera, Koffein, DHA-Omega-3 Fettsäuren, Extrakt von Gardenia-Stammzellen.

Vorzugsweise kann es sich bei den Wirkstoffen des erfindungsgemäßen Stoffgemisches um feuchtigkeitsspendende Substanzen sowie Öle und Fette, Salze, Emulgatoren und/oder Konservierungsstoffe handeln. Die lipophile Phase kann bevorzugt auf Kohlenwasserstoff basierende Paraffine und Glyceride umfassen. Vorteilhafterweise kommen diese Wirkstoffe als Bestandteile von Biomembranen vor oder können als Emulgatoren wirken, sodass die Creme eine gleichmäßige angenehme Konsistenz erhält. Damit das Stoffgemisch weiter über eine bessere Haltbarkeit und Konsistenz verfügt und dabei vorteilhafterweise ebenfalls ein angenehmes Hautgefühl durch das Auftragen des Stoffgemisches hervorruft, kann pflanzliches Fett, Kakaobutter, Mandelöl, Erdnussöl, Rizinusöl, Sojaöl, Wollwachs, Bienenwachs, Polysorbat 60, Rizinol-, 12-Hydroxystearin- und 11-Hydroxypalmitinsäure, Rizinusöl, Japanwachs, Carnaubawachs, Cetylpalmitat, Isopropylpalmitat, Jojobaöl, Squalen, Squalan, Cholesterin, Cholesterylsulfat, Phytosterole, Lanolin, Lanolinalkohole, Cetylstearylalkohol, Cetearyl Glucosid, Ceramide, Sphingolipide, Sphinganin, Sphingosin eingesetzt werden. Weiterhin können ebenfalls Polysorbate, Macrogolether, Fettalkoholsulfat sowie Eucerit, Sorbitanfettsäureester und Monoglyceride als weitere Emulgatoren wirken, die eine verbesserte Löslichkeit bewirken und ebenfalls ein angenehmes Hautgefühl des Stoffgemisches hervorrufen. Ferner können die Wirkstoffe Lidocain, Benzocain, Polidocanol, Isoprenalin, Crotamiton, Quinisocain, Meclozin, Cetirizin, Promethazin, Diphenhydramin, Chlophenoxamin, Doxylaamin, Pheniramin, Prompheniramin, Dexchlorpheniramin, Bamipin, Clemastin, Dimetinden, Mebhydrolin, Lorastadin, Oxatomid, Terfenadin, Astemizol, Triamcinolonacetonid, Bufexamac, Kamillenextrakt, Hamamelisextrakt, Gerbstoffe, Bisabolol, Acetylsalicylsäure, Allantoin durch ihre Eigenschaft als Oberflächenanästhetikum bzw. antiinflammatorisch wirkende Wirkstoffe ein angenehmes Hautgefühl verstärken und beispielsweise gereizte oder trockene Hautstellen vermindern. Sorbinsäure sowie Parabene können der Konservierung des Stoffgemisches und damit der längeren Haltbarkeit und somit ebenfalls einer längeren Verwendbarkeit dienen. Als Antioxidantien können Butylhydroxytoluol sowie α-Tocopherol eingesetzt werden, die die Oxidation von Inhaltsstoffen verhindern.

Ferner sind ebenfalls Wirkstoffe für den Schutz der Haut, wie insbesondere Triglyceride, Natriums, Kaliums, Magnesiums, Calciums, Zinks, Fluoride, Chloride, Sulfate, Phosphate, 2-Amino-ethylphosphate, Glycolate, Lactate, Citrate, Succinate, Malate, Fumarate, Monomethylfumarate, Monoethylfumarate, Tartrate denkbar.

Die Vitamine A, insbesondere Retinol und/oder Retinolsäure, Ascorbinsäure-2-Phosphat, Vitamin E, Vitamin D3, Vitamin B6/B12 und Vitamin B3 können vorteilhafterweise entgegen einer vorzeitigen Hautalterung wirken, indem beispielsweise die Teilung von Zellen und somit die Erneuerung der Haut gefördert werden. Weitere mögliche Vitamine und Provitamine können insbesondere Vitamin A- und B-Komplex, C, E, D sowie deren Derivate, insbesondere Vitamin A-Säure, Vitamin A-Acetat, Vitamin A-Palmitat, Vitamin C-Palmitat, Vitamin C-Phosphat, Vitamin E-Acetat, -Palmitat und -Linoleat, Alfacalcidol, Calcitriol, Colecalciferol, Ergocalciferol, Transcalcifediol, Calciprotriol, Calcifediol, Vitamin D3, β-Carotin, Panthenol, Pantothensäure, Biotin sein. Ebenfalls kann Folsäure eine zuverlässige und regelmäßige Zellteilung veranlassen. Acetyl-L-Carnitin kann ferner eine Zellerneuerung positiv beeinflussen. Carnosin sowie Coenzym Q10 und Carnitin können eine erhöhte Elastizität der Haut bewirken. Alpha-Liponsäure sowie Palmitoyl-Lysin-Threonin-Threonin-Lysin-Serin (pal-KTTS) und 2-Dimethylaminoethanol (DMAE) können ferner einer Faltenbildung entgegenwirken. Ebenfalls können hierfür die Wirkstoffe Argireline, Antarcticine und lipophilisierte Peptide, Kollagen, insbesondere Atelokollagen, Desamidokollagen eingesetzt werden,

Curcumin sowie DHA-Omega-3 Fettsäure können einen natürlichen Schutzfilm der Haut aufbauen und diese vor Umwelteinflüssen schützen. Aloe Vera wie auch Grüner-Tee-Extrakt, Algenextrakt, Echinaceaextrakt, Arnikaextrakt, Gurkenextrakt, Hopfenextrakt, Karottenextrakt, Kleieextrakt, Hamamelisextrakt, Hefeextrakt, Gingko-Biloba-Extrakt können ferner als ein reichhaltiger Feuchtigkeitslieferant der Hautzellen dienen. Feuchtigkeit, insbesondere Flüssigkeiten werden so dem Wirkort zugeführt und verbleiben an diesem. Damit entsteht ein glattes und weiches Hautbild. Weitere Feuchtigkeitslieferanten können ebenfalls Glykol, Propylenglykol, Butylenglykol, Hexylenglykol, Glycerin, Inositol (Inosit), Sorbitol (Sorbit), Mannit, Palatinit, Maltodextrin, Dextrin, Cyclodextrin, Glucose, Fructose, Lactose, Mannose, Galaktose sowie andere Saccharide, besonders bevorzugt können Glycerin, Propylenglykol, Pentylenglykol und/oder Harnstoff sein.

Weiterhin können vorzugsweise lipophile Wirkstoffe sowie UV-Filter verwendet werden. Bevorzugt können die UV-A- und UV-B-Filter 1-(4'-Isopropylphenyl)-3-phenylpropan-1.3-dion, 1-(4-tert.-Butylphenyl)-3-(4-methoxyphenyl)-propan-1.3-dion, 2.4-2.4-Dihydroxybenzophenon, 2-Hydroxy-4-methoxybenzophenon, 2.2'-Dihydroxy-4.4'-dimethoxybenzophe-non, 4-(Bis-(2-hydroxypropyl)-amino)-benzoesäureethylester, 4-Dimethylaminobenzoesäure-2-ethylhexylester, 3-(4-Methylbenzyliden)-bornan-2-on, 3-Benzyliden-bornan-2-on, 4-Methoxyzimtsäure-2-ethylhexylester, 4-Methoxyzimtsäurepropylester, 4-Methoxyzimt-säureisoamylester, 2-Cyano-3.3-diphenylacrylsäure-2-ethylhexylester, 3.3.5-Trimethylcylohexyl-salicylat, 4-Isopropylbenzylsalicylat, Salicylsäureoctylester, Salicylsäure-2-ethylhexylester2.4.6-Trianilino-p-(carbo-2'-ethylhexyl-1'-oxi)-1,3.5-triazin, Methylen-bis-benzotriazolyltetramethylbutylphenol, Ethylhexyloxyphenol Methoxyphenyl Triazin, 3-imidazol-4-ylacrylsäureethylester, Zinkoxid, Titandioxid einzeln oder in jeglicher Kombination verwendet werden. UV-Filter verleihen am Wirkort einen Schutz gegen UV-Strahlen, die die Zellen dauerhaft schädigen können. Ein ebenes Hautbild kann damit länger erhalten bleiben.

Ebenfalls ist es denkbar, dass Pigmente und Wirkstoffe mit abdeckender Wirkung, insbesondere Silikone, Magnesiumoxid, Magnesiumcarbonat, Magnesiumaluminiumsilikat, Magnesiumstearat, Magnesiumisostearat, Talkum, Calciumcarbonat, Zinkoxid, Zinkcarbonat, Zinkstearat, Zinklaurat, Titandioxid, Eisenoxid, Eisenhexacyanoferrat, Wismutoxychlorid, Aluminiumoxid, Alumosilikate, Siliciumdioxid, Aerosil, Bornitrid, Glimmer und Gesteinsmehle, Dihydroxyaceton in dem erfindungsgemäßen Stoffgemisch enthalten sind. Vorteilhafterweise können damit Hautunebenheiten sowie lokale Hautschäden und Hautanomalien optisch abgeschwächt, insbesondere kaschiert werden.

Bevorzugt kann das Stoffgemisch zumindest einen der folgenden Inhaltsstoffe enthalten: Wasser, Glycerin, Cetearylalkohol, Capric/Caprylic Triglycerid, Squalan, Butylenglycol, Dimeticon (Polydimethylsiloxan), Butyrospermum Parkii (Shea) Butter, Isopropylpalmitat, Cetearyl Glucosid, Polymethylsilsequioxan, Natrium Hylaluronate Crosspolymer Gardenia Florida Blütenextrakt, Phosphorsäure-Cetylester, Cetylphosphat (Kaliumsalz), Tocopheryl Acetate, Astragalus Membranaceus Wurzelextrakt, Talkum, Parfum (Duft), Hydroxyethyl Acrylate/Sodium Acryloyldimethyl Taurat Copolymer, Xanthan-Gummi, L-Ascorbyltetraisopalmita, Zitronensäure, Hydrogen Dimethicon, 1,2-Dihydroxypentan (Pentylenglykol), POLYSORBAT 60, Aluminiumhydroxyd, Edetinsäure Dinatriumsalz bzw. EDTA (Ethylendiamintetraacetat) Disodium, 2-Phenoxyethanol, Ethylhexylglycerin bzw. 3-(2-Ethylhexyloxy)-1,2-propandiol, Cl 77891 (Titanoxid)

Dimeticon ist eine langkettige organische Siliciumverbindung, die die Oberflächenspannung von beispielsweise Gasblasen senkt und diese dadurch auflöst. Dimeticon verhilft der Haut zu einem glatten, ebenen und weichen Erscheinungsbild. Sheabutter enthält ferner Fettsäuren, insbesondere Ölsäure, Stearinsäure, Linolensäure und Palmitinsäure. Weiterhin enthält Sheabutter Allantoin und weist damit eine entzündungshemmende, zellregenerierende und wundheilende Eigenschaft auf, die insbesondere Hautirritationen entgegenwirkt. Polymethylsilsequioxan verringert ferner die Transparenz und die Lichtdurchlässigkeit von kosmetischen Mitteln. Natrium Hyaluronate Crosspolymer bewahrt die Feuchtigkeit der Haut bzw. des kosmetischen Mittels und wirkt hautpflegend. Gardenia Florida Blütenextrakt wirken antioxidativ sowie leicht antientzündlich und fördert die Bildung neuer Hautzellen. Parfum und Duftstoffe verleihen einen angenehmen Duft des Stoffgemisches. Phosphorsäure-Cetylester, Cetylphosphat (Kaliumsalz) fördert die Bildung fein verteilter Mischungen (Emulsionen) nicht mischbarer Flüssigkeiten durch Änderung der Grenzflächenspannung und wird somit bevorzugt als Tensid eingesetzte, welches die Grenzflächenspannung von Stoffgemischen verringert und zu einer gleichmäßigen Verteilung bei der Anwendung verwendbar ist. Bei Hydroxyethyl Acrylate/Sodium Acryloyldimethyl Taurat Copolyer handelt es sich bevorzugt um N-Acyl-Derivate, Ester oder Salze des Taurins (2-Aminoethansulfonsäure). Acrylate sind Polymere oder Copolymere basierend auf Acrylsäure und/oder anderen Alkylacrylaten (Acrylsäureestern). Methacrylate sind Polymere oder Copolymere basierend auf Methacrylsäure und/oder deren Estern. Hydroxyethyl Acrylate/Sodium Acryloyldimethyl Taurat Copolymer ermöglicht dabei die freie Beweglichkeit von Feststoffen und verhindert so das Zusammenbacken pulverförmiger kosmetischer Mittel und wirkt bevorzugt zur Unterstützung der Emulsionsbildung und verbessert die Emulsionsbeständigkeit und -haltbarkeit. Weiterhin verringert es die Transparenz und die Lichtdurchlässigkeit von Stoffgemischen und erhöht oder verringert deren Viskosität. Xanthan-HGummi gewährleistet die Kohäsion von pulver- und puderhaltigen Stoffgemischen, fördert die Bildung fein verteilter Mischungen (Emulsionen) ansonsten nicht mischbarer Flüssigkeiten durch Änderung ihrer Grenzflächenspannung und unterstützt die Emulsionsbildung und verbessert die Emulsionsbeständigkeit und - haltbarkeit. Ferner ist Xanthan-Gummi dazu geeignet einer flüssigen Zubereitung die Konsistenz eines Gels zu verleihen, verringert die Grenzflächenspannung von Stoffgemischen und erhöht oder verringert die Viskosität kosmetischer Mittel. L-Ascorbyltetraisopalmitat hemmt Reaktionen, die durch Sauerstoff gefördert werden und verhindert so eine Oxidation und das Ranzigwerden von Inhaltsstoffen. Hydrogen Dimethicion bildet beim Auftragen einen zusammenhängenden Film auf Haut, Haar oder Nägeln. 1,2-Dihydroxypentan (Pentylenglykol) dient als Lösungsmittel und löst andere Stoffe auf bzw. hält diese in Lösung. Aluminiumhydroxid wird bevorzugt zum Ummanteln mineralischer Sonnenschutzfilter eingesetzt. Edetinsäure Dinatriumsalz bzw. EDTA (Ethylendiamintetraacetat) Disodium reagiert und bildet Komplexe mit Metallionen, welche die Stabilität und/oder das Aussehen von Stoffgemischen beeinflussen könnten. Ferner erhöht oder verringert es die Viskosität von Stoffgemischen. 2-Phenoxyethanol hemmt in erster Linie die Entwicklung von Mikroorganismen in Stoffgemischen. Ethylhexylglycerin bzw. 3-(2-Ethylhexyloxy)-1,2-propandiol wirkt bevorzugt hautpflegend. Titanoxid wirkt ferner als Sonnenschutz.

Darüber hinaus ist es ebenfalls denkbar medizinische Wirkstoffe für die Behandlung verschiedener Krankheiten wie Infektionskrankheiten der Haut, Entzündungen, insbesondere der Haut, Dermatosen, Hauttumore, physikalische und/oder chemische Schädigungen der Haut, vererbliche Hautkrankheiten und/oder Pigmentstörungen in dem erfindungsgemäßen Stoffgemisch zu verwenden.

Ferner wird die Aufgabe durch ein Verfahren zur Herstellung eines Glykosaminoglykan enthaltenen Stoffgemisches zur Anwendung in einem Verfahren für eine dermatologische Behandlung der Haut gelöst. Das erfindungsgemäße Verfahren bringt dabei die gleichen Vorteile mit sich, wie sie ausführlich in Bezug auf das erfindungsgemäße Stoffgemisch beschrieben worden sind.

Erfindungsgemäß umfasst das Verfahren zumindest einen der folgenden Schritte: Lösen des Glykosaminoglykans in Flüssigkeit. Dabei kann insbesondere destilliertes Wasser verwendet werden. Das Lösen des Glykosaminoglykans kann dabei derart erfolgen, dass abschließend eine Konzentration von ungefähr 0,2 Gew.-% bis ungefähr 5 Gew.-% erreicht werden, bevorzugt eine Konzentration von ungefähr 1 Gew.-% bis ungefähr 2 Gew.-%. Als weiteren Schritt kann das Verfahren das Einstellen des pH-Wertes umfassen. Der pH-Wert kann dabei auf einen Wert zwischen 4 und 7, insbesondere auf den pH-Wert von ungefähr 5 eingestellt werden. Alle Wirkstoffe können allein oder in einer entsprechend sinnvollen Kombination in dem erfindungsgemäßen Stoffgemisch enthalten sein. Sofern eine Einstellung des pH-Wertes aufgrund sauer reagierender Wirkstoffe notwendig ist, kann dies mittels anorganischer Basen wie z. B. Natriumhydroxid, Kaliumhydroxid, oder organischer Basen, wie z. B. Triethylamin oder Triethanolamin erfolgen. Als weiteren Schritt kann ein Erhitzen durchgeführt werden. Das Erhitzen kann dabei insbesondere mit einer Temperatur von ungefähr 40°C bis ungefähr 100°C erfolgen, insbesondere bei 80°C. Ein Erhitzen kann dabei vorteilhafterweise dazu dienen, die Wirkstoffe sowie Additive zu lösen. Bevorzugt kann das Erhitzen während des Lösens des Glykosaminoglykans in Flüssigkeit erfolgen. Dabei kann insbesondere destilliertes Wasser verwendet werden. Als nächsten Schritt kann das Verfahren ein Abkühlen des Stoffgemisches umfassen. Ein Abkühlen kann dabei insbesondere auf eine Temperatur von ungefähr 0°C bis ungefähr 30°C, bevorzugt auf eine Temperatur von ungefähr 10°C erfolgen. Ein Abkühlen kann vorteilhafterweise dazu dienen, eine homogene Konsistenz des Stoffgemisches zu erzeugen. Als weiteren Schritt kann ein Lösen des Astragalosid IV in Flüssigkeit vorgesehen sein. Dabei ist hervorzuheben, dass Astragalosid IV sehr stark lipophil ist und sich nicht in Wasser lösen lässt. Es wird eine Emulsion mittels mehrphasiger Zubereitung hergestellt. Damit Astragalosid IV nicht durch das Lösungsmittel inaktiviert oder beschädigt wird und auch keine Lösungsmittel verwenden werden, die negative Einflüsse auf die Haut haben könnten, erfolgt das Auflösen und Aufnehmen von Astragalosid IV in mittelkettigen Fettsäuren (MCT) aus Kokos- bzw. Palmkernöl. Es erfolgt eine schonende Mischung von MCTs und Astragalosid IV in einem Verhältnis von 2:1 mittels Homogenisiermischer. Das Lösen des Astragalosid IV kann dabei derart erfolgen, dass abschließend eine Konzentration von ungefähr 0,1 Gew.-% bis ungefähr 5 Gew.-% erreicht werden, bevorzugt eine Konzentration von ungefähr 0,1 Gew.%. Als weiteren Schritt kann ein Erhitzen durchgeführt werden. Das Erhitzen kann dabei insbesondere mit einer Temperatur von ungefähr 20°C bis ungefähr 60°C erfolgen, insbesondere bei 40°C. Ein Erhitzen kann dabei vorteilhafterweise dazu dienen, die Wirkstoffe sowie Additive zu lösen aber gleichzeitig die Wirksamkeit des Astragalosid IV nicht zu beeinträchtigen. Bevorzugt kann das Erhitzen während des Lösens des Astragalosid IV in Flüssigkeit erfolgen. Als nächsten Schritt kann das Verfahren ein Abkühlen des Stoffgemisches umfassen. Ein Abkühlen kann dabei insbesondere auf eine Temperatur von ungefähr 0°C bis ungefähr 30°C, bevorzugt auf eine Temperatur von ungefähr 10°C erfolgen. Ein Abkühlen dient vorteilhafterweise dazu, eine homogene Konsistenz des Stoffgemisches zu erzeugen. Ferner ist es denkbar, dass alle Schritte in jeglicher Reihenfolge und auch mehrfach nacheinander durchgeführt werden können.

Gemäß einem weiteren Aspekt der Erfindung ist eine Verwendung eines Glykosaminoglykan enthaltenes Stoffgemisch beansprucht. Die erfindungsgemäße Verwendung bringt dabei die gleichen Vorteile mit sich wie sie bereits ausführlich in Bezug auf das erfindungsgemäße Verfahren sowie das erfindungsgemäße Stoffgemisch beschrieben worden sind.

Erfindungsgemäß ist die Verwendung eines Glykosaminoglykan enthaltenes Stoffgemisch zur dermatologischen Behandlung der Haut topisch anwendbar. Diese Anwendung kann vorteilhafterweise auf der Haut stattfinden. Unter einer topischen Anwendung kann dabei die Anwendung von Wirkstoffen an dem Ort (Wirkort) verstanden werden, an dem sie wirken sollen. Die topische Anwendung kann sich dabei auf eine Anwendung auf einer Oberfläche, vorliegend insbesondere auf der Hautoberfläche, beziehen. Eine topische Anwendung kann dabei vorzugsweise lokal erfolgen. Vorteilhafterweise können die Wirkstoffe nur an dem Wirkort wirken, an dem sie benötigt werden, wobei andere Wirkorte, insbesondere Hautpartien, die gegebenenfalls andere Wirkstoffe benötigen, nicht beeinflusst werden. Dabei können Nebenwirkungen an anderen Stellen, insbesondere der Haut oder sogar systemische Nebenwirkungen vermieden werden. Weiterhin hat Astragalosid IV eine sehr eingeschränkte orale Bioverfügbarkeit und bietet sich für eine topische Behandlung an. Ferner ist eine bessere lokale Dosierung am Wirkort ermöglicht.

Weiterhin ist es denkbar, dass das Stoffgemisch eine Creme und/oder eine Lotion und/oder eine Salbe und/oder ein Spray und/oder ein Puder und/oder eine Emulsion und/oder ein Gel ist. Bei einer Creme kann es sich dabei um ein Stoffgemisch aus Anteilen von Fett und Flüssigkeit handeln, die miteinander vermischt sind. Vorzugsweise können dafür Emulgatoren eingesetzt werden, die die Bestandteile vermischt halten. Vorteilhafterweise können Cremes schnell in die Haut einziehen. Bei Lotionen kann es sich ebenfalls um Stoffgemische aus Fett und Flüssigkeit handeln, wobei der Flüssigkeitsanteil höher als der einer Creme sein kann. Lotionen können dabei noch schneller in die Haut einziehen und hinterlassen keinen Film auf der Haut. Auch Gele ziehen schnell in die Haut ein und hinterlassen keinen Film auf der Haut. Gele haben zudem vorteilhafterweise eine kühlende Wirkung. Salben können Stoffgemische aus Fett und Flüssigkeit sein, wobei der Fettanteil sehr hoch ausfallen kann. Diese können insbesondere für trockene Hautpartien gut geeignet sein und oftmals einen schützenden und pflegenden Film auf der Haut hinterlassen. Ferner sind Feststoffe wie beispielsweise Puder denkbar, die um eine gleichmäßige Auftragung erreichen zu können, jedoch lediglich lokal angewendet werden. Eine Auftragung in Puderform ist ebenfalls als Paste denkbar, wobei dem Puder vorteilhafterweise in dieser Form Fette zur besseren Auftragungsmöglichkeit beigemischt werden können. Ferner ist ein Stoffgemisch in Form einer Emulsion denkbar. Eine Emulsion ist ein Stoffgemisch zweier normalerweise nicht mischbarer Flüssigkeiten, ohne sichtbare Entmischung. Die eine Flüssigkeit liegt dabei in kleinen Tröpfchen verteilt in der anderen Flüssigkeit vor. Eine Auftragung verschiedener Wirkstoffe in einem Anwendungsschritt wird dabei ermöglicht.

Insbesondere ist eine Anwendung im Gesicht und weiteren sichtbaren Hautpartien mit erhöhtem Voralterungspotential (UV Alterung) denkbar. Eine besondere Pflege dieser Hautpartien kann oftmals besonders wirksam sein, da diese stets den Umwelteinflüssen ausgesetzt und wenig bedeckt sind. Durch eine topische Anwendung im Gesicht kann einer Faltenentwicklung vorgebeugt werden und bereits bestehende Falten können reduziert werden. Ebenfalls denkbar ist eine Verwendung, die eine Hautalterung vermindert. Dabei können insbesondere eine intrinsische und/oder eine extrinsische Hautalterung vermindert werden.

Weiterhin ist es möglich, dass die Auftragung auf die Haut alle ungefähr 0,5 Std. bis ungefähr 48 Std. erfolgt, insbesondere alle ungefähr 1 Std. bis ungefähr 24 Std. und bevorzugt alle ungefähr 2 Std. bis ungefähr 12 Std.. Damit kann das Stoffgemisch bevorzugt zweimal innerhalb von 24 Std. aufgetragen werden. Je häufiger die Anwendung erfolgt, desto größer ist die Konzentration des Stoffgemisches, welches pro Fläche an dem Wirkort einwirken kann. Da die Wirkung des Stoffgemisches, bevorzugt topisch und damit lokal erfolgt, ist eine mehrmalige Anwendung pro Tag vorteilhaft, um die Wirkstoffkonzentration am Wirkort hochzuhalten und eine ausreichende Versorgung der Haut zu gewährleisten, wodurch ein gleichmäßigeres und ebenes Hautbild entsteht. Insbesondere abends, bevorzugt zwischen 18:00 und 22:00 Uhr kann eine Auftragung des Stoffgemisches auf die Haut vorteilhaft sein, da die natürliche Regeneration der Haut über Nacht somit weiter unterstützt wird.

Es ist ebenfalls denkbar, bei einer Verwendung jeweils ungefähr 0,1 g bis ungefähr 10 g pro Auftragung auf die Haut aufzutragen, insbesondere jeweils ungefähr 0,5 g bis ungefähr 5 g bevorzugt ungefähr 1 g bis ungefähr 3 g. Weiterhin ist es vorteilhaft, wenn eine Sättigung der Aufnahmekapazität am Wirkort erreicht werden kann. Insbesondere bei einer Konzentration des Stoffgemisches von ungefähr 1 g bis ungefähr 3 g kann die Aufnahmekapazität der Haut erreicht sein. Dabei ist es ferner vorteilhaft, wenn ein angenehmes Hautbild bzw. ein angenehmes Gefühl auf der Haut entstehen soll, dass das Stoffgemisch nahezu vollständig in die Haut einziehen kann und keine Rückstände auf der Haut verbleiben, so dass die Haut keinen feuchten, fettenden Eindruck hinterlässt. Bei Verwendungsformen, die einen Film oder Rückstände, insbesondere zur Pflege oder zum Schutz der Haut am Wirkort hinterlassen, können vorzugsweise Pigmente oder Stoffe mit abdeckender Wirkung beigemischt werden, so dass das Erscheinungsbild der Haut einen optisch ebenen und straffen Eindruck hinterlassen kann.

Weitere, die Erfindung verbessernde Maßnahmen ergeben sich aus der nachfolgenden Beschreibung zu einem Ausführungsbeispiel der Erfindung, welches in den Figuren schematisch dargestellt ist. Sämtliche aus den Ansprüchen, der Beschreibung oder der Zeichnung hervorgehende Merkmale und/oder Vorteile, einschließlich konstruktiver Einzelheiten, räumliche Anordnungen und Verfahrensschritte, können sowohl für sich als auch in den verschiedensten Kombinationen erfindungswesentlich sein. Dabei ist zu beachten, dass die Figuren nur beschreibenden Charakter haben und nicht dazu gedacht sind, die Erfindung in irgendeiner Form einzuschränken. Es zeigt:

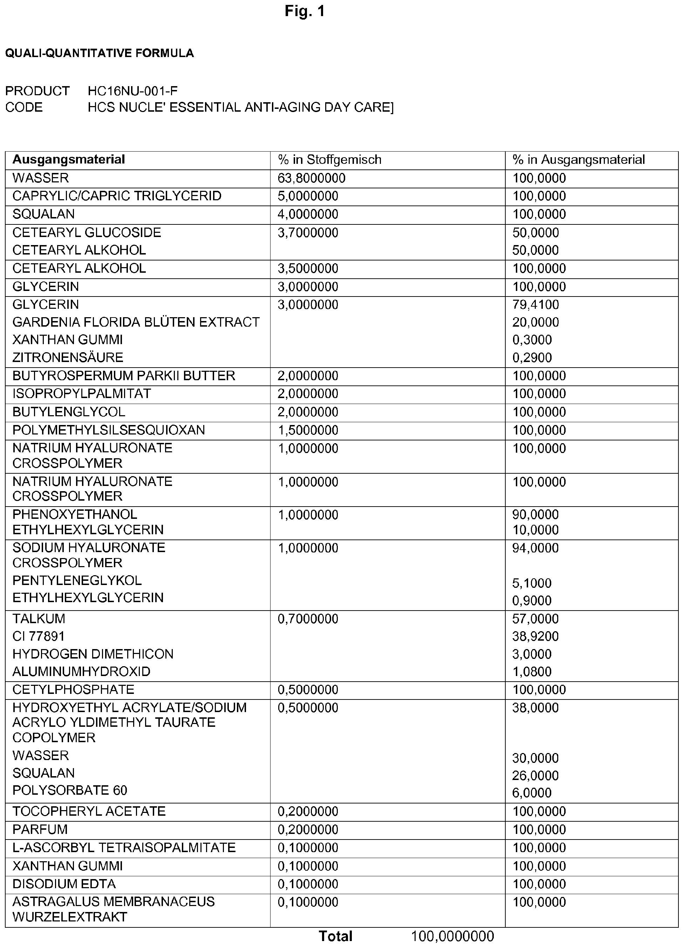

- Fig 1: Tabellarische Übersicht des Stoffgemisches

Figur 1 zeigt eine tabellarische Übersicht eines Stoffgemisches in % bezogen auf die Endkonzentration des Stoffgemisches. Es ist ein beispielhaftes Ausführungsbeispiel einer Zusammensetzung eines Stoffgemisches dargestellt, welches exemplarische die Anteile einzelner Inhaltsstoffe zeigt. Bei dem dargestellten Ausführungsbeispiel handelt es sich bevorzugt um eine Hautcreme, insbesondere eine Creme zur Anwendung im Gesicht.

## Patentansprüche

1. Verfahren zur Behandlung der Haut, umfassend ein Glykosaminoglykan enthaltenes Stoffgemisch zur Anwendung in einem Verfahren für die dermatologische Behandlung der Haut,
**dadurch gekennzeichnet,**
**dass** Astragalosid IV enthalten ist,
wobei das Glykosaminogylkan im Wesentlichen Cross-Chain-Hyaluronketten aufweist, wobei das Glykosaminoglykan eine Konzentration von 0,2 Gew.-% bis 5 Gew.-% und das Astragalosid IV eine Konzentration von 0,1 Gew.-% bis 5 Gew.-% aufweist.

2. Verfahren gemäß Anspruch 1,
**dadurch gekennzeichnet,**
**dass** das Glykosaminoglykan eine Konzentration von 0,4 Gew.-% bis 2 Gew.-% und besonders bevorzugt eine Konzentration von 1 Gew.-% bis 2 Gew.-% aufweist.

3. Verfahren gemäß einem der vorhergehenden Ansprüche,
**dadurch gekennzeichnet,**
**dass** das Astragalosid IV eine Konzentration von 0,1 Gew.-% bis 2 Gew.-% und besonders bevorzugt eine Konzentration von 0,1 Gew.-% aufweist.

4. Verfahren gemäß einem der vorhergehenden Ansprüche,
**dadurch gekennzeichnet,**
**dass** zumindest einer der folgenden weiteren Additive vorgesehen ist: Paraffin, Glycerid, pflanzliches Fett, Kakaobutter, Mandelöl, Erdnussöl, Rizinusöl, Sojaöl, Wollwachs, Bienenwachs, Polysorbat 60, Rizinol-, 12-Hydroxystearin- und 11-Hydroxypalmitinsäure, Rizinusöl, Japanwachs, Carnaubawachs, Cetylpalmitat, Isopropylpalimtat, Jojobaöl, Squalen, Squalan, Cholesterin, Cholesterylsulfat, Phytosterol, Lanolin, Cetearylalkohol, Cetearyl Glucosid, Lanolinalkohol, Ceramide, Sphingolipid, Sphinganin, Sphingosin, Polysorbat, Macrogolether, Fettalkoholsulfat, Eucerit, Sorbitanfettsäureester, Monoglycerid, Sorbinsäure, Paraben, Butylhydroxytoluol, α-Tocopherol, Resveratrol, Vitamin A Säure, insbesondere Retinol und/oder Retinolsäure, Ascorbinsäure-2-Phosphat, Vitamin E, Vitamin D, Vitamin B, β-Carotin, Panthenol, Pantothensäure, Biotin, Folsäure, Acetyl-L-Carnitin, Alpha-Liponsäure, Carnosin, Palmitoyl-Lysin-Threonin-Threonin-Lysin-Serin (pal-KTTS), 2-Dimethylaminoethanol (DMAE), Carnitin, Coenzym Q 10, Curcumin, Aloe Vera, Grüner-Tee-Extrakt, Algenextrakt, Echinaceaextrakt, Arnikaextrakt, Gurkenextrakt, Hopfenextrakt, Karottenextrakt, Kleieextrakt, Hamamelisextrakt, Hefeextrakt, Gingko-Bbiloba-Extrakt, Glykol, Propylenglykol, Butylenglykol, Hexylenglykol, Glycerin, Inositol, Sorbitol, Mannit, Palatinit, Maltodextrin, Dextrin, Cyclodextrin, Glucose, Fructose, Lactose, Mannose, Galaktose, Glycerin, Propylenglykol, Pentylenglykol, Harnstoff, DHA-Omega-3 Fettsäuren, Triglycerid, Natrium, Kalium, Magnesium, Calcium, Zink, Fluoride, Chlorid, Sulfat, Phosphat, 2-Amino-ethylphosphat, Glycolat, Lactat, Citrat, Succinat, Malat, Fumarat, Monomethylfumarat, Monoethylfumarat, Tartrat, Argirelin, Antarcticin, lipophilisiertes Peptid, Kollagen, Magnesiumoxid, Magnesiumcarbonat, Magnesiumaluminiumsilikat, Magnesiumstearat, Magnesiumisostearat, Talkum, Calciumcarbonat, Zinkoxid, Zinkcarbonat, Zinkstearat, Zinklaurat, Titandioxid, Eisenoxid, Eisenhexacyanoferrat, Wismutoxychlorid, Aluminiumoxid, Alumosilikat, Siliciumdioxid, Aerosil, Bornitrid, Glimmer, Dihydroxyaceton.

5. Verfahren zur Herstellung eines Glykosaminoglykan enthaltenen Stoffgemisches, gemäß einem der Ansprüche 1 bis 4, zur Anwendung in einem Verfahren für die dermatologische Behandlung der Haut, umfassend zumindest einen der folgenden Schritte:
- Lösen des Glykosaminoglykans in einer Flüssigkeit insbesondere in einer Konzentration von 0,2 Gew.-% bis 5 Gew.-%,
- Einstellen des pH-Wertes, insbesondere auf einen pH-Wert von 4 bis 7,
- Erhitzen des Stoffgemisches, insbesondere auf eine Temperatur von 40°C bis 100°C,
- Abkühlen des Stoffgemisches, insbesondere auf eine Temperatur von 0°C bis 30°C,
- Lösen des Astragalosid IV in mittelkettigen Triglyzeriden in einem Verhältnis von 1:2 mit einer Konzentration von 0,1 Gew.-% bis 5 Gew.-%,
- Erhitzen des Stoffgemisches, insbesondere auf eine Temperatur von 20°C bis 60°C,
- Abkühlen des Stoffgemisches, insbesondere auf eine Temperatur von 0°C bis 30°C.

6. Verwendung eines Glykosaminoglykan enthaltenen Stoffgemisches, gemäß einem der Ansprüche 1 bis 4, zur dermatologischen Behandlung der Haut,
**dadurch gekennzeichnet,**
**dass** das Stoffgemisch topisch anwendbar ist, insbesondere auf der Haut.

7. Verwendung gemäß Anspruch 6,
**dadurch gekennzeichnet,**
**dass** das Stoffgemisch eine Creme und/oder eine Lotion und/oder eine Salbe und/oder ein Spray und/oder ein Puder und/oder eine Emulsion und/oder ein Gel ist, insbesondere zur Anwendung im Gesicht.

8. Verwendung gemäß einem der Ansprüche 6 oder 7,
**dadurch gekennzeichnet,**
**dass** eine Hautalterung vermindert wird, insbesondere, dass eine intrinsische und/oder eine extrinsische Hautalterung vermindert wird.

9. Verwendung gemäß einem der Ansprüche 6 bis 8,
**dadurch gekennzeichnet,**
**dass** eine Auftragung auf die Haut alle 0,5 Std. bis 48 Std., insbesondere alle 1 Std. bis 24 Std., bevorzugt alle 2 Std. bis 12 Std. erfolgt.

10. Verwendung gemäß einem der Ansprüche 6 bis 9,
**dadurch gekennzeichnet,**
**dass** jeweils 0,1 g bis 10 g pro Auftragung auf die Haut aufgetragen werden, insbesondere, dass jeweils 0,5 g bis 5 g, bevorzugt, dass jeweils 1 g bis 3 g auf der Haut aufgetragen werden.

## Claims

1. A method of treating the skin comprising a glycosaminoglycan-containing mixture of substances for use in a method of dermatological treatment of the skin,
**characterized in that**
astragaloside IV is included,
wherein the glycosaminoglycan has substantially cross-chain hyaluron chains, wherein the glycosaminoglycan has a concentration of from 0.2% to 5% by weight and the astragaloside IV has a concentration of from 0.1% to 5% by weight.

2. The method according to claim 1,
**characterized in that**
the glycosaminoglycan has a concentration of 0.4% to 2% by weight and, particularly preferably, a concentration of 1% to 2% by weight.

3. A method according to any one of the preceding claims,
**characterized in that**
the astragaloside IV has a concentration of 0.1 wt.% to 2 wt.% and particularly preferably a concentration of 0.1 wt.%.

4. A method according to any one of the preceding claims,
**characterized in that**
at least one of the following further additives is provided: Kerosene, glyceride, vegetable fat, cocoa butter, almond oil, peanut oil, castor oil, soybean oil, lanolin, beeswax, polysorbate 60, ricinoleic, 12-hydroxystearic and 11hydroxypalmitic acids, castor oil, Japan wax, carnauba wax, cetyl palmitate, isopropyl palimate, jojoba oil, squalene, squalane, cholesterol, cholesteryl sulfate, phytosterol, lanolin, cetearyl alcohol, Cetearyl glucoside, lanolin alcohol, ceramides, sphingolipid, sphinganine, sphingosine, polysorbate, macrogol ether, fatty alcohol sulfate, euceritol, sorbitan fatty acid ester, monoglyceride, sorbic acid, paraben, butylhydroxytoluene, α-tocopherol, Resveratrol, vitamin A acid, especially retinol and/or retinoic acid, ascorbic acid-2-phosphate, vitamin E, vitamin D, vitamin B, β-carotene, panthenol, pantothenic acid, biotin, folic acid, acetyl-L-carnitine, alpha-lipoic acid, Carnosine, palmitoyl-lysine-threonine-lysine-serine (pal-KTTS), 2-dimethylaminoethanol (DMAE), carnitine, coenzyme Q 10, curcumin, aloe vera, green tea extract, algae extract, echinacea extract, arnica extract, cucumber extract, hops extract, Carrot extract, Bran extract, Hamamelis extract, Yeast extract, Gingko Biloba extract, Glycol, Propylene glycol, Butylene glycol, Hexylene glycol, Glycerin, Inositol, Sorbitol, Mannitol, Palatinitol, Maltodextrin, Dextrin, Cyclodextrin, Glucose, Fructose, Lactose, Mannose, Galactose, Glycerin, Propylene Glycol, Pentylene Glycol, Urea, DHA Omega-3 Fatty Acids, Triglyceride, Sodium, Potassium, Magnesium, Calcium, Zinc, Fluoride, Chloride, Sulfate, Phosphate, 2-Aminoethyl Phosphate, Glycolate, Lactate, citrate, succinate, malate, fumarate, monomethyl fumarate, monoethyl fumarate, tartrate, argirelin, antarcticin, lipophilized peptide, collagen, magnesium oxide, magnesium carbonate, magnesium aluminum silicate, magnesium stearate, magnesium isostearate, Talc, calcium carbonate, zinc oxide, zinc carbonate, zinc stearate, zinc laurate, titanium dioxide, iron oxide, ferric ferricyanide, bismuth oxychloride, aluminum oxide, aluminosilicate, silicon dioxide, aerosil, boron nitride, mica, dihydroxyacetone.

5. A method of preparing a glycosaminoglycan-containing substance mixture, according to any one of claims 1 to 4, for use in a method for dermatological treatment of the skin, comprising at least one of the following steps:
- Dissolving the glycosaminoglycan in a liquid, in particular at a concentration of 0.2% to 5% by weight,
- Adjusting the pH value, in particular to a pH value of 4 to 7,
- Heating the mixture of substances, in particular to a temperature of 40°C to 100°C,
- Cooling the mixture of substances, in particular to a temperature of 0°C to 30°C,
- Dissolving astragaloside IV in medium chain triglycerides at a ratio of 1:2 with a concentration of 0.1 wt% to 5 wt%,
- Heating the mixture of substances, in particular to a temperature of 20°C to 60°C,
- Cooling the mixture of substances, in particular to a temperature of 0°C to 30°C.

6. Use of a glycosaminoglycan-containing substance mixture, according to any one of claims 1 to 4, for the dermatological treatment of the skin,
**characterized in that**
the substance mixture is topically applicable, especially on the skin.

7. Use according to claim 6,
**characterized in that**
the mixture of substances is a cream and/or a lotion and/or an ointment and/or a spray and/or a powder and/or an emulsion and/or a gel, in particular for application to the face.

8. Use according to any one of claims 6 or 7,
**characterized in that**
skin aging is reduced, in particular that intrinsic and/or extrinsic skin aging is reduced.

9. Use according to any one of claims 6 to 8,
**characterized in that**
application to the skin takes place every 0.5 to 48 hours, in particular every 1 to 24 hours, preferably every 2 to 12 hours.

10. Use according to any one of claims 6 to 9,
**characterized in that**
in each case 0.1 g to 10 g per application are applied to the skin, in particular that in each case 0.5 g to 5 g, preferably that in each case 1 g to 3 g are applied to the skin.

## Revendications

1. Procédé de traitement de la peau comprenant un mélange de substances contenant un glycosaminoglycane destiné à être utilisé dans un procédé de traitement dermatologique de la peau,
**caractérisé en ce que**
l'astragaloside IV est contenu,
dans lequel le glycosaminoglycane comprend essentiellement des cha nes hyaluroniques croisées, le glycosaminoglycane ayant une concentration de 0,2 % en poids à 5 % en poids et l'astragaloside IV ayant une concentration de 0,1 % en poids à 5 % en poids.

2. Procédé selon la revendication 1,
**caractérisé en ce que**
le glycosaminoglycane présente une concentration de 0,4 % en poids à 2 % en poids et de manière particulièrement préférée une concentration de 1 % en poids à 2 % en poids.

3. Procédé selon l'une quelconque des revendications précédentes,
**caractérisé en ce que**
l'astragaloside IV présente une concentration de 0,1 % en poids à 2 % en poids et de manière particulièrement préférée une concentration de 0,1 % en poids.

4. Procédé selon l'une quelconque des revendications précédentes,
**caractérisé en ce qu'**
il est prévu au moins l'un des additifs supplémentaires suivants : Paraffine, glycéride, graisse végétale, beurre de cacao, huile d'amande, huile d'arachide, huile de ricin, huile de soja, cire de laine, cire d'abeille, polysorbate 60, acides ricinoléique, 12-hydroxystéarique et 11hydroxypalmitique, huile de ricin, cire du Japon, cire de carnauba, palmitate de cétyle, palimate d'isopropyle, huile de jojoba, squalène, cholestérol, sulfate de cholestérol, phytostérol, lanoline, alcool cétéarylique, Cetearyl Glucosid, alcool de lanoline, céramides, sphingolipide, sphinganine, sphingosine, polysorbate, macrogol éther, sulfate d'alcool gras, euceritol, ester d'acide gras de sorbitan, monoglycéride, acide sorbique, paraben, butylhydroxytoluène, α-tocophérol, Resvératrol, vitamine A acide, en particulier rétinol et/ou acide rétinoïque, acide ascorbique-2-phosphate, vitamine E, vitamine D, vitamine B, β-carotène, panthénol, acide pantothénique, biotine, acide folique, acétyl-L-carnitine, acide alpha-lipoïque, Carnosine, palmitoyl-lysine-thréonine-thréonine-lysine-sérine (pal-KTTS), 2-diméthylaminoéthanol (DMAE), carnitine, coenzyme Q 10, curcumine, aloe vera, extrait de thé vert, extrait d'algue, extrait d'échinacée, extrait d'arnica, extrait de concombre, extrait de houblon, Extrait de carotte, extrait de son, extrait d'hamamélis, extrait de levure, extrait de gingko biloba, glycol, propylène glycol, butylène glycol, hexylène glycol, glycérine, inositol, sorbitol, mannitol, palatinitol, maltodextrine, dextrine, cyclodextrine, glucose, Fructose, lactose, mannose, galactose, glycérol, propylèneglycol, pentylèneglycol, urée, acides gras oméga-3 DHA, triglycéride, sodium, potassium, magnésium, calcium, zinc, fluorures, chlorure, sulfate, phosphate, 2-aminoéthylphosphate, glycolate, Lactate, citrate, succinate, malate, fumarate, monométhylfumarate, monoéthylfumarate, tartrate, argireline, antarcticine, peptide lipophilisé, collagène, oxyde de magnésium, carbonate de magnésium, silicate de magnésium et d'aluminium, stéarate de magnésium, isostéarate de magnésium, Talc, carbonate de calcium, oxyde de zinc, carbonate de zinc, stéarate de zinc, laurate de zinc, dioxyde de titane, oxyde de fer, hexacyanoferrate de fer, oxychlorure de bismuth, oxyde d'aluminium, aluminosilicate, dioxyde de silicium, aérosil, nitrure de bore, mica, dihydroxyacétone.

5. Procédé de préparation d'un mélange de substances contenant du glycosaminoglycane, selon l'une quelconque des revendications 1 à 4, destiné à être utilisé dans un procédé de traitement dermatologique de la peau, comprenant au moins l'une des étapes suivantes :
- Dissoudre le glycosaminoglycane dans un liquide, en particulier à une concentration de 0,2 % en poids à 5 % en poids,
- Ajuster le pH, en particulier à une valeur comprise entre 4 et 7,
- Chauffage du mélange de substances, en particulier à une température de 40°C à 100°C,
- Refroidissement du mélange de substances, en particulier à une température de 0°C à 30°C,
- Dissolution de l'astragaloside IV dans des triglycérides à chaîne moyenne dans un rapport de 1:2 avec une concentration de 0,1 % à 5 % en poids,
- Chauffage du mélange de substances, en particulier à une température de 20°C à 60°C,
- Refroidissement du mélange de substances, en particulier à une température de 0°C à 30°C.

6. Utilisation d'un mélange de substances contenant du glycosaminoglycane, selon l'une des revendications 1 à 4, pour le traitement dermatologique de la peau,
**caractérisé en ce que**
le mélange de substances est applicable par voie topique, en particulier sur la peau.

7. Utilisation selon la revendication 6,
**caractérisé en ce que**
le mélange de substances est une crème et/ou une lotion et/ou une pommade et/ou un spray et/ou une poudre et/ou une émulsion et/ou un gel, en particulier pour l'application sur le visage.

8. Utilisation selon l'une des revendications 6 ou 7,
**caractérisé en ce qu'**
un vieillissement de la peau est diminué, en particulier qu'un vieillissement intrinsèque et/ou extrinsèque de la peau est diminué.

9. Utilisation selon l'une quelconque des revendications 6 à 8,
**caractérisé en ce qu'**
une application sur la peau est effectuée toutes les 0,5 h à 48 h, en particulier toutes les 1 h à 24 h, de préférence toutes les 2 h à 12 h.

10. Utilisation selon l'une quelconque des revendications 6 à 9,
**caractérisé en ce que**
l'on applique respectivement de 0,1 g à 10 g par application sur la peau, en particulier **en ce que** l'on applique respectivement de 0,5 g à 5 g, de préférence respectivement de 1 g à 3 g sur la peau.
